# EUROPÄISCHE PATENTSCHRIFT

(11) **EP 1 007 671 B1**
(45) Veröffentlichungstag und Bekanntmachung des Hinweises auf die Patenterteilung: **13.02.2002**
(21) Anmeldenummer: 98921434.1
(22) Anmeldetag: 06.04.1998
(51) Int. Cl.: C12N 15/12, C12Q 1/68, C12N 5/10, A61K 48/00, A61K 38/17, G01N 33/50, C07K 14/47, C07K 16/18

(54) **FANCONI-GEN II**
FANCONI-GEN II
GENE II DE FANCONI

(30) Priorität: 07.04.1997 EP 97105688
(43) Veröffentlichungstag der Anmeldung: 14.06.2000
(73) Patentinhaber: Roche Diagnostics GmbH, 68305 Mannheim (DE)
(72) Erfinder: KUBBIES, Manfred, D-82377 Penzberg (DE); MACHL, Andreas, D-94469 Deggendorf (DE); PLANITZER, Simone, D-12309 Berlin (DE)
(74) Vertreter: Schreiner, Siegfried, Dr.
(86) Internationale Anmeldenummer: EP9801994
(87) Internationale Veröffentlichungsnummer: WO9845428

(56) Entgegenhaltungen:
- WO-A-93/22435
- WO-A-98/23775
- EMBL, Zugangsnummer Y10275, H. sapiens mRNA for L-3-phosphoserine phosphatase, Kreiert 15-3-97, XP002077007
- EMBL EST, Zugangsnummer W44613, Sequenzkennzeichen zc29d04.r1, 27-Mai-1996, Soares senescent fibroblasts NbHSF Homo sapiens cDNA Klone 323719 5' XP002040596
- EMBL EST, Zugangsnummer g1664579, Sequenzkennzeichen zn68f05.s1, 09-Nov-96, Stratagene HeLa cell s3 937216 Homo sapiens cDNA Klone 563361 3' XP002040597
- EMBL EST, Zugangsnummer W44574, Sequenzkennzeichen zc29d04.s1, 27-Mai-1996, Soares senescent fibroblasts NbHSF Homo sapiens cDNA Klone 323719 3' XP002018662
- COLLET J -F ET AL: "HUMAN L-3-PHOSPHOSERINE PHOSPHATASE: SEQUENCE, EXPRESSION AND EVIDENCE FOR A PHOSPHOENZYME INTERMEDIATE" FEBS LETTERS, Bd. 408, Nr. 3, 26. Mai 1997, Seiten 281-284, XP002056286
- PLANITZER, A. ET AL.: "Identification of a novel c-DNA overexpressed in Fanconi's anemia fibroblasts partially homologous to a putative L-3-phosphoserine-phosphatase" GENE, Bd. 210, Nr. 2, 14. April 1998, Seiten 297-306, XP002076751 AMSTERDAM NL

### SEQUENZPROTOKOLL

(1) ALLGEMEINE ANGABEN:
   (i) ANMELDER:
      (A) NAME: Boehringer Mannheim GmbH
      (B) STRASSE: Sandhofer Str. 112-132
      (C) ORT: Mannheim
      (E) LAND: Deutschland
      (F) POSTLEITZAHL: 68305
   (ii) BEZEICHNUNG DER ERFINDUNG: Fanconi-Gen II
   (iii) ANZAHL DER SEQUENZEN: 2
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 1026 Basenpaare
      (B) ART: Nucleotid
      (C) STRANGFORM: beides
      (D) TOPOLOGIE: linear
   (ix) MERKMAL:
      (A) NAME/SCHLÜSSEL: CDS
      (B) LAGE:256..924
   (ix) MERKMAL:
      (A) NAME/SCHLÜSSEL: CDS
      (B) LAGE:430..924
   (xi) SEQUENZBESCHREIBUNG: SEQ ID NO: 1:
   TTTCACCGTC TAGAGGCATA AGAGGTGAGC CCGTGCTCTT CAGCGGAGAA GATCCCCTAC      60
   CTGGCCGCCG GCCACTTTCT GTGGGCCGTG GGGTCCTCAA GGAGACGGCC CTTGGGCTCA     120
   GGGGCTGCGT TTCCACACGC GCCTTTCCCA GGGCTCCCGC GCCCGTTCCT GCCTGGCCGC     180
   CGGCCGCTCC AACAGCAGCA CAAGGCGGGA CTCAGAACCG GCGTTCAGGG CCGCCAGCGG     240
   CCGCGAGGCC CTGAG ATG AGG CTC CAA AGA CCC CGA CAG GCC CCG GCG GGT     291
                    Met Arg Leu Gln Arg Pro Arg Gln Ala Pro Ala Gly
                      1               5                  10
   GGG AGG CGC GCG CCC CGG GGC GGG CGG GGC TCC CCC TAC CGG CCA GAC      339
   Gly Arg Arg Ala Pro Arg Gly Gly Arg Gly Ser Pro Tyr Arg Pro Asp
            15                  20                  25
   CCG GGG AGA GGC GCG CGG AGG CTG CGA AGG TTC CAG AAG GGC GGG GAG       387
   Pro Gly Arg Gly Ala Arg Arg Leu Arg Arg Phe Gln Lys Gly Gly Glu
        30                  35                  40
   GGG GCG CCG CGC GCT GAC CCT CCC TGG GCA CCG CTG GGG ACG ATG GCG       435
   Gly Ala Pro Arg Ala Asp Pro Pro Trp Ala Pro Leu Gly Thr Met Ala
    45                  50                  55                  60
   CTG CTC GCC TTG CTG CTG GTC GTG GCC CTA CCG CGG GTG TGG ACA GAC       483
   Leu Leu Ala Leu Leu Leu Val Val Ala Leu Pro Arg Val Trp Thr Asp
                    65                  70                  75
   GCC AAC CYG ACT GCG AGA CAA CGA GAT CCA SAG GAC TCC CAG CGA ACG       531
   Ala Asn Xaa Thr Ala Arg Gln Arg Asp Pro Xaa Asp Ser Gln Arg Thr
                80                  85                  90
   GAC GAG GGT GAC AAT AGA GTG TGG TGT CAT GTT TGT GAG AGA GAA AAC       579
   Asp Glu Gly Asp Asn Arg Val Trp Cys His Val Cys Glu Arg Glu Asn
            95                 100                 105
   ACT TTC GAG TGC CAG AAC CCA AGG AGG TGC AAA TGG ACA GAG CCA TAC       627
   Thr Phe Glu Cys Gln Asn Pro Arg Arg Cys Lys Trp Thr Glu Pro Tyr
       110                 115                 120
   TGC GTT ATA GCG GCC GTG AAA ATA TTT CCA CGT TTT TTC ATG GTT GCG       675
   Cys Val Ile Ala Ala Val Lys Ile Phe Pro Arg Phe Phe Met Val Ala
   125                 130                 135                 140
   AAG CAG TGC TCC GCT GGT TGT GCA GCG ATG GAG AGA CCC AAG CCA GAG       723
   Lys Gln Cys Ser Ala Gly Cys Ala Ala Met Glu Arg Pro Lys Pro Glu
                   145                 150                 155
   GAG AAG CGG TTT CTC CTG GAA GAG CCC ATG CCC TTC TTT TAC CTC AAG       771
   Glu Lys Arg Phe Leu Leu Glu Glu Pro Met Pro Phe Phe Tyr Leu Lys
               160                 165                 170
   TGT TGT AAA ATT CGC TAC TGC AAT TTA GAG GGG CCA CCT ATC AAC TCA       819
   Cys Cys Lys Ile Arg Tyr Cys Asn Leu Glu Gly Pro Pro Ile Asn Ser
           175                 180                 185
   TCA GTG TTC AAA GAA TAT GCT GGG AGC ATG GGT GAG AGC TGT GGT GGG       867
   Ser Val Phe Lys Glu Tyr Ala Gly Ser Met Gly Glu Ser Cys Gly Gly
       190                 195                 200
   CTG TGG CTG GCC ATC CTC CTG CTG CTG GCC TCC ATT GCA GCC GGC CTC       915
   Leu Trp Leu Ala Ile Leu Leu Leu Leu Ala Ser Ile Ala Ala Gly Leu
   205                 210                 215                 220
   AGC CTG TCT TGAGCCACGG GACTGCCACA GACTGAGCCT TCCGGAGCAT               964
   Ser Leu Ser
   GGACTCGCTC CAGACCGTTG TCACCTGTTG CATTAAACTT GTTTTCTGTT GAAAAAAAAA    1024
   AA                                                                   1026
(2) ANGABEN ZU SEQ ID NO: 2:
   (i) SEQUENZKENNZEICHEN:
      (A) LÄNGE: 223 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Arg Leu Gln Arg Pro Arg Gln Ala Pro Ala Gly Gly Arg Arg Ala
    1               5                  10                  15
   Pro Arg Gly Gly Arg Gly Ser Pro Tyr Arg Pro Asp Pro Gly Arg Gly
               20                  25                  30
   Ala Arg Arg Leu Arg Arg Phe Gln Lys Gly Gly Glu Gly Ala Pro Arg
           35                  40                  45
   Ala Asp Pro Pro Trp Ala Pro Leu Gly Thr Met Ala Leu Leu Ala Leu
       50                  55                  60
   Leu Leu Val Val Ala Leu Pro Arg Val Trp Thr Asp Ala Asn Xaa Thr
   65                  70                  75                  80
   Ala Arg Gln Arg Asp Pro Xaa Asp Ser Gln Arg Thr Asp Glu Gly Asp
                   85                  90                  95
   Asn Arg Val Trp Cys His Val Cys Glu Arg Glu Asn Thr Phe Glu Cys
               100                 105                 110
   Gln Asn Pro Arg Arg Cys Lys Trp Thr Glu Pro Tyr Cys Val Ile Ala
           115                 120                 125
   Ala Val Lys Ile Phe Pro Arg Phe Phe Met Val Ala Lys Gln Cys Ser
       130                 135                 140
   Ala Gly Cys Ala Ala Met Glu Arg Pro Lys Pro Glu Glu Lys Arg Phe
   145                 150                 155                 160
   Leu Leu Glu Glu Pro Met Pro Phe Phe Tyr Leu Lys Cys Cys Lys Ile
                   165                 170                 175
   Arg Tyr Cys Asn Leu Glu Gly Pro Pro Ile Asn Ser Ser Val Phe Lys
               180                 185                 190
   Glu Tyr Ala Gly Ser Met Gly Glu Ser Cys Gly Gly Leu Trp Leu Ala
           195                 200                 205
   Ile Leu Leu Leu Leu Ala Ser Ile Ala Ala Gly Leu Ser Leu Ser
       210                 215                 220

## Beschreibung

Die vorliegende Erfindung betrifft ein pathophysiologisch relevantes Fanconi-Anämie (FA)-assoziiertes Gen, ein davon codiertes Polypeptid, einen gegen das Polypeptid gerichteten Antikörper sowie die pharmazeutische Anwendung der Nucleinsäure, des Polypeptids und des Antikörpers.

FA ist eine seltene genetische Erkrankung, die durch progressive Pancytopenie, kongenitale Abnormitäten und ein erhöhtes Risiko für Tumorerkrankungen gekennzeichnet ist (Glanz und Fraser, J.Med.Genet. 19 (1982) 412-416; Auerbach und Allen, Cancer Genet.Cytogenet. 51 (1991) 1-12). Sie tritt bei etwa einer von 300.000 Personen auf.

Obwohl die molekulare Basis der Erkrankung unbekannt ist, liefert die Hypersensitivität gegenüber der clastogenen Wirkung von DNA-Quervernetzungsmitteln einen guten Marker für den FA-Genotyp (Auerbach und Wolmann, Nature 261 (1976), 494-496). Zellen aus FA-Patienten zeigen multiple Chromatidbrüche und - austausche nach Kontakt mit Mitomycin C (MMC) oder Diepoxybutan (DEB) in Konzentrationen, die eine geringe clastogene Wirkung auf normale Zellen haben (Sasaki und Tonomura, Cancer Res. 33 (1973), 1829-1836 und Auerbach, Exp. Hematol. 21 (1993), 731). Nach Inkubation von FA-Lymphozyten und Fibroblasten mit MMC ist ein Defekt in der G2-Phase des Zellzyklus ersichtlich, der sich sowohl in einer Verzögerung des G2-Phasenübergangs als auch in einem vollständigen Arrest äußert (Kubbies et al., Am.J.Hum.Genet. 37 (1985), 1022; Hoehn et al., Fanconi Anemia, Clinical, Cytogenic and Experimental Aspects (1989), Springer Verlag Berlin-Heidelberg; Seyschab et al., Blood 85 (1995), 2233-2237).

Gegenwärtig sind innerhalb der FA-Population mindestens 5 verschiedene Komplementationsgruppen bekannt (Duckworth-Rysiecki et al., Somatic Cell Mol.Genet. 11 (1985), 35; Strathdee et al., Nature 356 (1992), 763; Joenje et al., Blood 86 (1995), 2156-2160). Die Gene für die Komplementationsgruppen A und C wurden vor kurzem beschrieben (Strathdee et al., Nature 356 (1992), 763; Lo Ten Fol et al., Nature Genet. 14 (1996), 320-323); WO93/22435; Pronk et al., Nature Genet. 11 (1995), 338-340), aber die Art der molekularen Wirkungsweise der FA-A und FA-C Proteine sind noch unbekannt (Gavish et al., Am. J. Hum. Genet. 53 (1993), 685; Yamashita et al., Proc. Natl. Acad. Sci. USA 91 (1994), 6712; Youssoufian et al., J. Biol. Chem. 270 (1995), 9876-9882). Weiterhin wurde für die Komplementationsgruppe FAD die chromosomale Lokalisierung angegeben (Whitney et al., Nature Genet. 11 (1995), 341-343).

Die Aufgabe der vorliegenden Erfindung bestand darin, neue Gene zu identifizieren, die in der DNA-Regulationskaskade beteiligt sind (z.B. Zellzyklusstörungen, DNA-Reparatur, Tumorgenese/Tumorprogression) und die mit dem pathophysiologischen Phänotyp der Fanconi-Anämie assoziiert sein können.

Die vorliegende Erfindung beschreibt die Identifizierung, Klonierung und Charakterisierung eines Gens, das als Fanconi-Gen II bezeichnet wird und für zwei neue Polypeptide codiert. Gefunden wurde diese Gensequenz unter Verwendung der Differential-Display-Technik (Liang und Pardee, Science 257 (1992), 967-971) im Vergleich von Normal-Fibroblasten und FA-Fibroblasten. Das Fanconi-Gen II wird nicht in FA-Fibroblasten aber in Normal-Fibroblasten exprimiert. Das Fanconi-Gen II, die davon codierten Polypeptide sowie gegen die Polypeptide gerichtete Antikörper sind als diagnostische, therapeutische oder präventive Mittel für Erkrankungen geeignet, die mit Störungen des Zellzyklus, der Zellaktivierung, der Zellzyklusprogression, der DNA-Reparatur, Cytopenien, Turmorgenese und Tumorprogression direkt oder indirekt assoziiert sind.

Ein Gegenstand der vorliegenden Erfindung ist eine Nucleinsäure, ausgewählt aus der Gruppe bestehend aus
(a) der in SEQ ID NO:1 dargestellten Nucleotidsäure oder einem Protein-codierenden Abschnitt davon,
(b) einer der Nukleinsäuren aus (a) im Rahmen der Degeneration des genetischen Codes entsprechenden Nucleotidsequenz,
(c) einer komplementären Nucleotidsäure hierzu,
(d) einer mit den Sequenzen aus (a) oder/und (b) unter stringenten Bedingungen hybridisierenden Antisense-Nucleotidsäure einer Länge von bis zu 50 Nucleotiden
mit der Maßgabe, daß die Nucleinsäure verschieden ist von den Nucleotidsäuren mit den Zugangsnummern W44613, W44574 und g1664579, angegeben in der EMBL EST Datenbank.

In der Datenbank EMBL EST sind drei cDNA-Sequenzen angegeben, W44613, W44574, g1664579, 1996, die Abschnitte der in SEQ ID NO. 1 angegebenen Nukleotidsequenz enthalten. Diese Sequenzen sind nicht Gegenstand der Erfindung. Sie offenbaren weder die vollständige erfindungsgemäße Nukleinsäuresequenz, noch einen funktionellen Protein-codierenden Abschnitt davon, da durch das Fehlen eines Startcodons in allen drei Sequenzen kein offener Leserahmen offenbart ist. Weiterhin weisen die drei Sequenzen keine 5'-UTRs auf und enthalten Deletionen, welche Verschiebungen des Leserahmens mit sich bringen. Außerdem wird für die drei oben genannten Sequenzen keine biologische Funktion offenbart.

Die in SEQ ID NO. 1 dargestellte Nucleotidsequenz enthält zwei offene Leserahmen, die Polypeptide mit einer Länge von 223 Aminosäuren und 165 Aminosäuren entsprechen. Diese Polypeptide reichen von Aminosäure 1 bis 223 bzw. von Aminosäure 59 bis 223 der in SEQ ID NO. 2 dargestellten Aminosäuresequenz.

In SEQ ID No. 1 ist ein Nucleotid Y, d.h. C oder T an Position 491 und ein Nucleotid S, d.h. C oder G an Position 514.

Neben der in SEQ ID NO. 1 gezeigten Nucleotidsequenz und einer dieser Sequenz im Rahmen der Degeneration des genetischen Codes entsprechenden Nucleotidsequenz umfaßt die vorliegende Erfindung auch noch eine Nucleotidsequenz, die mit einer der zuvor genannten Sequenzen unter stringenten Bedingungen hybridisiert. Der Begriff "stringente Hybridisierung" gemäß vorliegender Erfindung wird wie bei Sambrook et al. (Molecular Cloning. A Laboratory Manual, Cold Spring Harbor Laboratory Press (1989), 1.101-1.104) verwendet. Vorzugsweise spricht man von einer stringenten Hyb ridisierunbg, wenn nach Waschen für eine Stunde mit 1 X SSC und 0,1 % SDS bei 50°C, vorzugsweise bei 55°C, besonders bevorzugt bei 62°C und am meisten bevorzugt bei 68°C, insbesondere für eine Stunde in 0,2 X SSC und 0,1 % SDS bei 55°C, vorzugsweise bei 55°C, besonders bevorzugt bei 62°C und am meisten bevorzugt bei 68°C noch ein positives Hybridisierungssignal beobachtet wird. Eine unter derartigen Waschbedingungen mit der in SEQ ID NO. 1 gezeigten Nucleotidsequenz oder einer damit im Rahmen der Degeneration des genetischen Codes entsprechenden Nucleotidsequenz hybridisierende Nucleotidsequenz ist eine erfindungsgemäße Nucleotidsequenz.

Vorzugsweise ist die erfindungsgemäße Nucleotidsequenz eine DNA. Sie kann jedoch auch eine RNA oder ein Nucleinsäureanalogon wie eine peptidische Nucleinsäure umfassen. Die erfindungsgemäße Nucleinsäure umfaßt einen Protein-codierenden Abschnitt der in SEQ ID NO. 1 dargestellten Nucleotidsequenz oder einen vorzugsweise mindestens 20 nt. und besonders bevorzugt mindestens 50 nt. langen Abschnitt davon.

Ein weiterer Gegenstand der vorliegenden Erfindung sind die von einer Nucleinsäure wie oben angegebenen codierten Polypeptide. Die Polypeptide weisen vorzugsweise (a) die in SEQ ID NO. 2 dargestellte Aminosäuresequenz von Aminosäure 1 bis 223, (b) die in SEQ ID No. 2 dargestellte Aminosäuresequenz von Aminosäure 59 - 223 auf.

Erfindungsgemäße Nucleinsäuren sind vorzugsweise aus Säugern und insbesondere aus dem Menschen erhältlich. Sie können nach bekannten Techniken unter Verwendung kurzer Abschnitte der in SEQ ID NO. 1 gezeigten Nucleotidsequenz als Hybridisierungssonden oder/und Primer nach bekannten Methoden isoliert werden. Weiterhin können erfindungsgemäße Nucleinsäuren auch durch chemische Synthese hergestellt werden, wobei anstelle der üblichen Nucleotidbausteine gegebenenfalls auch modifizierte Nucleotidbausteine, z.B. 2'-O-alkylierte Nucleotidbausteine eingesetzt werden können. Nucleinsäuren, die teilweise oder vollständig aus modifizierten Nucleotidbausteinen bestehen, können beispielsweise als therapeutische Mittel, z.B. als Antisense-Nucleinsäuren oder Ribozyme eingesetzt werden.

Weiterhin umfaßt die Erfindung auch Nucleinsäureanaloga wie etwa peptidische Nucleinsäuren, deren Basensequenz einer erfindungsgemäßen Nucleinsäure entspricht.

Ein weiterer Gegenstand der vorliegenden Erfindung ist ein Vektor, der mindestens eine Kopie einer erfindungsgemäßen Nucleinsäure enthält. Dieser Vektor kann ein beliebiger prokaryontischer oder eurkaryontischer Vektor sein, auf dem sich die erfindungsgemäße DNA-Sequenz vorzugsweise unter Kontrolle eines Expressionssignals (Promotor, Operator, Enhancer etc.) befindet. Beispiele für prokaryontische Vektoren sind chromosomale Vektoren wie etwa Bakteriophagen und extrachromosomale Vektoren wie etwa Plasmide, wobei zirkuläre Plasmidvektoren besonders bevorzugt sind. Geeignete prokaryontische Vektoren sind z.B. bei Sambrook et al., Supra, Kapitel 1 - 4 beschrieben.

Besonders bevorzugt ist der erfindungsgemäße Vektor ein eukaryontischer Vektor, z.B. ein Hefevektor oder ein für höhere Zellen geeigneter Vektor (z.B. ein Plasmidvektor, viraler Vektor, Pflanzenvektor). Derartige Vektoren sind dem Fachmann auf dem Gebiet der Molekularbiologie geläufig, so daß hier nicht näher darauf eingegangen werden muß. Insbesondere wird in diesem Zusammenhang auf Sambrook et al., Supra, Kapitel 16 verwiesen.

Unter den Begriff "Variante" fallen sowohl natürlich vorkommende allelische Variationen oder Spleißvariationen der Fanconi-Polypeptide II sowie durch rekombinante DNA-Technologie (insbesondere in vitro-Mutagenese mit Hilfe von chemisch synthetisierten Oligonucleotiden) erzeugte Proteine, die hinsichtlich ihrer biologischen oder/und immunologischen Aktivität den in SEQ ID No. 2 dargestellten Proteinen im wesentlichen entsprechen. Ebenfalls fallen unter diesen Begriff auch chemisch modifizierte Polypeptide. Hierzu gehören Polypeptide, die an den Termini oder/und an reaktiven Aminosäureseitengruppen durch Acylierung, z.B. Acetylierung, oder Amidierung modifiziert sind.

Die Erfindung betrifft auch einen Vektor, der mindestens einen 20 Nucleotide langen Abschnitt der in SEQ ID No. 1 dargestellten Sequenz enthält. Vorzugsweise besitzt dieser Abschnitt eine Nucleotidsequenz, die aus dem Protein-codierenden Bereich der in SEQ ID NO. 1 dargestellten Sequenz oder einem für die Expression des Proteins wesentlichen Bereich stammt. Diese Nucleinsäuren eigenen sich besonders zur Herstellung von therapeutisch einsetzbaren Antisense-Nucleinsäuren, die vorzugsweise bis zu 50 Nucleotide lang sind.

Ein weiterer Gegenstand der vorliegenden Erfindung ist eine Zelle, die mit einer erfindungsgemäßen Nucleinsäure oder einem erfindungsgemäßen Vektor transformiert ist. Die Zelle kann sowohl eine eukaryontische als auch eine prokaryontische Zelle sein. Verfahren zur Transformation von Zellen mit Nucleinsäuren sind allgemeiner Stand der Technik und brauchen daher nicht näher erläutert zu werden. Beispiele für bevorzugte Zellen sind eukaryontische Zellen, insbesondere tierische und besonders bevorzugt Säugerzellen.

Ebenfalls ein Gegenstand der vorliegenden Erfindung ist die Verwendung des erfindungsgemäßen Polypeptids oder Fragmenten dieses Polypeptids als Immunogen zur Herstellung von Antikörpern. Die Herstellung von Antikörpern kann dabei auf übliche Weise durch Immunisierung von Versuchstieren mit dem vollständigen Polypeptid oder Fragmenten davon und anschließende Gewinnung der resultierenden polyklonalen Antiseren erfolgen. Nach der Methode von Köhler und Milstein oder deren Weiterentwicklungen können aus den Antikörper-produzierenden Zellen der Versuchstiere auf bekannte Weise durch Zellfusion monoklonale Antikörper erhalten werden. Ebenso können nach bekannten Methoden humane monoklonale Antikörper hergestellt werden.

Als Immunogen bevorzugt sind die rekombinanten Fanconi II-Proteine oder Peptidfragmente, insbesondere N- oder C-terminale Peptide hiervon.

Ein weiterer Gegenstand der vorliegenden Erfindung ist somit ein Antikörper gegen die Fanconi II-Proteine oder Varianten davon, vorzugsweise Antikörper, die keine Kreuzreaktion mit anderen Fanconi-assoziierten Proteinen wie etwa dem FAC-Protein zeigen. Besonders bevorzugt sind Antikörper gegen die gesamten Polypeptide oder gegen eine Peptidsequenz gerichtet, die den Aminosäuren 1-40, 59-120 oder 205-223 der in SEQ ID NO. 2 dargestellten Aminosäuresequenz entsprechen.

Die Bereitstellung der Fanconi II-Proteine, dafür codierender Nucleinsäuren und dagegen gerichteter Antikörper schaffen die Voraussetzung für eine gezielte Suche nach Effektoren dieser Proteine. Stoffe, die auf das erfindungsgemäße Polypeptid inhibitorisch oder aktivierend wirken, sind in der Lage, die durch die Polypeptide gesteuerten Zellfunktionen selektiv zu beeinflussen. Daher können sie bei der Therapie entsprechender Krankheitsbilder wie z.B. Cytopenien oder Tumore eingesetzt werden. Ein Gegenstand der Erfindung ist somit auch ein Verfahren zur Identifizierung von Effektoren der Fanconi II-Proteine, wobei man Zellen, die das Protein exprimieren, mit verschiedenen potentiellen Effektorsubstanzen, z.B. niedermolekularen Stoffen, in Kontakt bringt und die Zellen auf Veränderungen, z.B. zellaktivierende, zellinhibierende, zell-proliferative oder/und zellgenetische Veränderungen, analysiert. Auf diese Weise lassen sich auch Bindetargets der Fanconi II-Proteine identifizieren.

Bei Krankheitsbildern, die auf einen Ausfall der Fanconi II-Proteine zurückzuführen sind, kann eine gentherapeutische Behandlung erfolgen, welche die Übertragung einer für die Fanconi Proteine-II codierenden Nucleinsäuren mittels Vektoren, z.B. viralen Vektoren, in das entsprechende Zielgewebe umfaßt. Andererseits kann bei Krankheitsbildern, die auf eine unkontrollierte Expression der Fanconi-Proteine II zurückzuführen sind, eine gentherapeutische Behandlung erfolgen, welche zu einer Blockierung dieser Expression führt.

Die vorgestellten Ergebnisse schaffen überdies die Voraussetzung für eine gezielte Diagnostik von Krankheiten, die mit Veränderungen der Aktivität der Fanconi-Proteine II ursächlich oder indirekt verbunden sind. Diese Untersuchungen können mit Hilfe spezifischer Nucleinsäuresonden zum Nachweis auf Nucleinsäureebene, z.B. auf Gen- oder Transkriptebene, oder mit Hilfe von Antikörpern gegen die Fanconi-Proteine II zum Nachweis auf Polypeptidebene durchgeführt werden.

Die vorliegende Erfindung betrifft somit eine pharmazeutische Zusammensetzung, die als aktive Komponenten Nucleinsäuren, Vektoren, Zellen, Polypeptide und Antikörper wie zuvor angegeben enthält.

Die erfindungsgemäße pharmazeutische Zusammensetzung kann weiterhin pharmazeutisch übliche Träger-, Hilfs- oder/und Zusatzstoffe sowie gegebenenfalls weitere aktive Komponenten enthalten. Die pharmazeutische Zusammensetzung kann insbesondere zur Diagnostik, Therapie oder Prävention von Erkrankungen eingesetzt werden, die mit Störungen des Zellzyklus, der Zellaktivierung, der Zellzyklusprogression, der DNA-Reparatur sowie mit Cytopenien, der Tumorgenese oder/und der Tumorprogression assoziiert sind. Weiterhin kann die erfindungsgemäße Zusammensetzung auch zur Diagnostik einer Prädisposition für solche Erkrankungen bei Individuen, insbesondere bei der Diagnostik eines Risikos für Cytopenien oder/und Tumorerkrankungen eingesetzt werden.

Noch ein weiterer Gegenstand der vorliegenden Erfindung ist ein Verfahren zur Diagnostik der oben genannten Erkrankungen, wobei man einen Patienten oder eine aus einem Patienten stammende Probe, z.B. eine Probe einer Körperflüssigkeit oder eines Gewebes, mit einer erfindungsgemäßen pharmazeutischen Zusammensetzung in Kontakt bringt und die Nucleotidsequenz oder/und die Expression der erfindungsgemäßen Nucleinsäure qualitativ oder quantitativ bestimmt. Diese Bestimmungsmethoden können beispielsweise auf Nucleinsäureebene durch Verwendung von Nucleinsäure-Hybridisierungssonden oder über Reverse Transkription/PCR bzw. auf Proteinebene durch Antikörper nach cyto- oder histochemischen Methoden erfolgen. Besonders bevorzugt ist die Verwendung der pharmazeutischen Zusammensetzung als Marker für das Auftreten von Cytopenien, Tumoren oder anderen proliferations-assoziierten Erkrankungen oder einer Prädisposition für die genannten pathophysiologischen Veränderungen.

Die erfindungsgemäße pharmazeutische Zusammensetzung zur Therapie oder Prävention einer der zuvor genannten Erkrankungen geeignet.

Die Zusammensetzung enthält die aktive Komponente in einer gegen die Erkrankung wirksamen Menge. Spezifische Beispiele für pharmazeutische Zusammensetzungen, welche für therapeutische Zwecke geeignet sind, sind beispielsweise bispezifische Antikörper und Antikörper-Toxin- oder Antikörper-Enzymkonjugate. Weitere bevorzugte pharmazeutische Zusammensetzungen für therapeutische Zwecke sind Antisense-Nucleinsäuren, gentherapeutische Vektoren oder andere niedermolekulare Aktivatoren oder Inhibitoren.

Die Erfindung wird durch die folgenden Beispiele und Sequenzprotokolle näher erläutert. Es zeigen:
- SEQ ID NO. 1: eine Nucleotidsequenz, die für das Fanconi-Gen II codierende genetische Information enthält, wobei ein größerer offener Leserahmen von Nucleotid 256 - 924 und ein kleinerer offener Leserahmen von Nucleotid 430 - 924 reicht, und
- SEQ ID NO. 2: die Aminosäuresequenzen der offenen Leserahmen der in SEQ ID NO. 1 gezeigten Nucleotidsequenz, wobei die Aminosäuresequenz des größeren offenen Leserahmens von Aminosäure 1 - 223 und die Aminosäuresequenz des kleineren offenen Leserahmens von Aminosäure 59 - 223 reicht.

### BEISPIELE

### Beispiel 1:

### Kultivierung von Zellen

Primäre diploide humane Fibroblasten H94-38 und H94-17 wurden aus fötalem Lungengewebe isoliert und von D. Schindler (Universität Würzburg) zur Verfügung gestellt. Die H94-38 Zellen wurden durch Zellzyklusanalyse als Fanconi-Anämie-Phänotyp diagnostiziert und weisen eine verlängerte G2-Phase sowie einen Anstieg des G2-Phasenarrests bei Zugabe von MMC auf. Aus Komplementationsuntersuchungen geht hervor, daß die H94-38 Zellen nicht zu den Fanconi-Komplementationsgruppen A, B, C und D, sondern vermutlich zur Komplementationsgruppe E gehören. H94-17 Kontrollzellen zeigen keine erhöhte MMC-Sensitivität.

Die Zellen wurden bei 37°C mit 7% CO₂ und 95% Feuchtigkeit in MEM Medium mit Earle's Salzen (BRL, Gaithersburg MD, USA) unter Zusatz von 10% fötalem Kälberserum (Hyclone, Logan, UT, USA) kultiviert. Zur RNA-Präparation wurden die Zellen durch Serumentzug (0,1%) synchronisiert und nach 48 h mit 10% fötalem Kälberserum stimuliert. Nach weiteren 30 h waren die Zellen subkonfluent und konnten für die RNA-Isolierung geerntet werden.

Zur Analyse des Zellzyklusstatus in einem Proliferationsassay wurden nach einer Kultivierungsdauer von 30 h in BrdU-haltigem Medium Aliquots dieser Zellkulturen entnommen. Mittels einer hochauflösenden flußzytometrischen BrdU-Hoechst-Quenchingtechnik wurden die Anzahl von Zellen in den Zellzyklusphasen G0/G1, S und G2/M wie von Kubbies (in Radbruch, A. (ed.), Flow Cytometry and Cell Sorting, Springer Verlag Berlin-Heidelberg 1992, pp 75-85) beschrieben bestimmt.

### Beispiel 2 :

### mRNA Differential-Display

Für den mRNA Differential-Display wurde der RNA-Kit von Gen Hunter (Brookline, MA, USA) verwendet. Die Gesamt-RNA wurde aus synchronisierten Zellkulturen mit dem Tripure-Reagenz (Boehringer Mannheim GmbH, DE) gemäß der Vorschrift des Herstellers isoliert. Bis zur Verwendung wurde die RNA als Isopropanol-präzipitierte RNA-Pellets, bedeckt mit 70% Ethanol bei - 80°C aufbewahrt. DNAse I (Boehringer Mannheim GmbH) wurde zu 1-5 µg Gesamt-RNA in 1 X DNAse I Reaktionspuffer gegeben und bei 37°C für 30 min inkubiert.

Die RNA-Proben wurden durch Messung der Absorption bei 260 nm quantitativ bestimmt und auf einem Agarosegel analysiert. 0,2 µg Gesamt-RNA wurden für die reverse Transkription verwendet. Aus 1 x 10⁶ Fibroblasten wurden insgesamt 8 µg Gesamt-RNA isoliert.

Die reverse Transkription der RNA erfolgte in doppelten Ansätzen von jeweils 20 µl in 1 X reversem Transkriptionspuffer, jeweils 20 µM dNTPs und 2 µM jeweils der einbasigen Ankerprimer T₁₁A, T₁₁G oder T₁₁C. Die Lösung wurde 5 min auf 65°C erwärmt, 10 min auf 37°C abgekühlt und dann wurden 100 U Moloney Murine Leukemiavirus (MMLV) Reverse Transkriptase zugegeben. Nach Inkubation für eine Stunde bei 37°C wurde das Gemisch für 5 min auf 75°C erhitzt und dann bei -20°C aufbewahrt.

Die PCR wurde in einer Reaktionslösung durchgeführt, die 1/10 Volumen des Ansatzes zur reversen Transkription, 2 µM dNTPs, 0,2 µM des jeweiligen T₁₁N-Primers, 0,2 µM eines Primers mit willkürlich festgelegter Sequenz, 10 µCi α[³⁵S]dATP und 1 U Taq-DNA-Polymerase (Boehringer Mannheim GmbH) enthielt. Die PCR wurde in einem Perkin-Elmer 2400 Gene Amp. PCR-System für insgesamt 40 Zyklen bei 94°C 30 sec, 40°C 2 min, 72°C 30 sec und schließlich 72°C für 5 min durchgeführt. Es wurden verschiedene willkürliche Primer von Gen Hunter (13-mer mit Hind-III Restriktionsstelle), Operon (Allameda CA, USA) und Genosys (The Woodlands, TX, USA) (jeweils 10-mer Primer mit 60-70% GC-Gehalt) verwendet.

Die Proben wurden in Sequenziergel-Beladungspuffer bei 80°C für 2 min vor der Auftrennung auf einen 5-6% denaturierenden Polyacrylamid-Sequenziergel denaturiert. Für jede Probe wurden doppelte PCR Ansätze durchgeführt und auf dem gleichen Polyacrylamidgel nebeneinander aufgetrennt. Das getrocknete Gel wurde autoradiographisch auf differentiell exprimierte Gene analysiert.

Reproduzierbare Banden, die differenziell exprimierten Genen entsprechen, wurden aus dem Gel ausgeschnitten. Die cDNA wurde aus Gelstücken durch Kochen für 15 min in 100 µl sterilem Wasser eluiert. Die DNA im Überstand wurde durch Ethanolpräzipitation in Gegenwart von Glycogen gesammelt. Anschließend wurde die DNA zur Reamplifikation mit den entsprechenden Primern und PCR-Bedingungen wie oben angegeben verwendet, außer daß dNTP-Konzentrationen von 20 µM verwendet wurden und das Reaktionsgemisch keine Radioisotope enthielt.

Die auf diese Weise erhaltenen amplifizierten PCR-Fragmente wurden über ein Agarosegel aufgetrennt und durch Zentrifugation des entsprechenden Gelstücks in einem 0,45 µm Millipore Durapore Membranschlauch eluiert. Die Proben wurden für die Northern-Analyse bei -20°C aufbewahrt.

Auf diese Weise wurden mit 106 verschiedenen Primerkombinationen insgesamt 60 Banden, davon 43 reamplifizierte Banden erhalten, die in FA-Zellen und Kontrollzellen differentiell exprimierten Genen entsprechen. Diese differentielle Expression war reproduzierbar.

### Beispiel 3 :

### Northern-Analyse

Die Northern Blot Analyse wurde gemäß Standardprozeduren gemäß Sambrook et al. (1989), Supra durchgeführt. Die Nucleinsäuren wurden auf positiv-geladene Nylonmembranen (Boehringer Mannheim GmbH) durch nach unten gerichteten Kapillartransfer mit 10 X SSC transferiert und quervernetzt.

Spezifische Sonden wurden direkt aus dem PCR Reamplifikationsansatz durch Markierung mit dem Hi-Prime Markierungskit (Boehringer Mannheim GmbH) unter Verwendung von Hexamerprimern mit willkürlicher Sequenz markiert. Die Abtrennung freier Nucleotide erfolgte unter Verwendung von G50 Sephadex Schleudersäulen (Boehringer Mannheim GmbH).

Die auf diese Weise erzeugten Sonden wurden gegen Gesamt-RNA hybridisiert. Nach Hybridisierung für 16 - 20 h bei 42°C wurde der Filter 2 mal in 1 X SSC, 0,1% SDS bei Raumtemperatur für 15 min und anschließend in 1 X SSC 0,1% SDS bei 50°C für 1 h gewaschen. Dann wurden die Membranen autoradiographisch untersucht.

Die Northern-Analyse ergab eine differentielle Expression für ein etwa 1020 bp langes PCR-Fragment.

Northern-Analysen in Zellkultur- und Gewebeproben ergaben in Kontrollfibroblasten eine dominante Bande mit einer Länge von ca. 1 kb und oftmals eine weitere Bande oder singulär auftretende Bande mit einer Länge von ca. 700 bp, die vielleicht auf eine Spleißvariante zurückgeführt werden kann. In den untersuchten FA-Fibroblasten wurden diese Banden nicht gefunden. In der Tumorzellinie Hela wurden beide Varianten stark exprimiert. In anderen Tumorzellinien (z. B. Raji oder K562) wurde keine Expression gefunden. In Embryonalfibroblasten aus dem Knorpel der Augenpigmentschale wurde nur die größere Bande gefunden.

### Beispiel 4:

### Charakterisierung von DD-PCR-Fragmenten, die differentiell exprimierten mRNA-Spezies entsprechen

PCR-Fragmente, die sich im Northern Blot als differentiell erwiesen, wurden mittels dem TA Cloning Kit (INVITROGEN) nach Vorschrift des Herstellers in den Vektor pCR™2.1 ligiert und der E.coli Stamm INVαF' mit diesem Konstrukt transformiert. Klone, die das Plasmid aus differenziellem Fragment und Vektor enthielten, wurden gemäß Standardprozeduren nach Sambrook et al. (1989, Cold Spring Harbor University Press, Cold Spring Harbor, N.Y.) kultiviert und die Plasmide isoliert.

Der 5'-Bereich der gefundenen Nucleotidsequenz wurde mittels einer modifizierten NewRACE Technik (Frohmann, M. A. (1994). On Beyond Classic RACE. PCR Methods Applic 4:40-58.) amplifiziert. Hierzu wurde in Gegenwart von 20 % PEG/DMSO (1:1, w/v) an das 5' Ende der Gesamt-RNA der Kontrollzellen ein DNA/RNA-Oligonucleotid (5'-GTAAAACGACGGCCAGTAAAGCACTCTCCAGCCTCTCA CCGCrArArA-3') ligiert und die modifizierte RNA mit dem genspezifischen Primer SP1 (5'-AACAGAAAACAAGTTTAATGCAACAGGTGA-3') revers transkribiert. Das gesamte cDNA Transkript wurde mit einem Primer (5'-CACTCTCCAGCCTCTCACCGCAAA-3') sowie dem genspezifischen Primer SB2 (5'-GCTGAGGCC GGCTGCAATGGA-3') amplifiziert und wie oben beschrieben in den Vektor pCR™2.1 ligiert und sequenziert. Die Zugehörigkeit beider Fragmente zur selben mRNA wurde durch einen überlappenden Bereich von 380 Nucleotiden mit identischer Basenfolge und eine PCR mit außen liegenden Primern am 5'Ende des RACE Fragments (5'-TTTCACCGTCTAGAGGCATAAGAGG-3') und am 3'Ende des Differential Display Fragments (5'-AACAGAAAACAAGTTTAATGCAA CAGGTGA-3') mit dem Ergebnis der Sequenz des Fanconi Gens II erwiesen. Die dadurch erhaltene cDNA des Fanconi Gens II wurde wie oben beschrieben in den Vektor pCR™2.1 ligiert und sequenziert. Mittels Northern Blot Analyse wie in Beispiel 3 beschrieben wurde die differenzielle Expression der gesamten Fanconi Gen II mRNA nachgewiesen.

### Beispiel 5:

### Herstellung eines Expressionskonstrukts und Expression

Exemplarisch wird hier die Expression der langen Variante des FA-II Gens beschrieben. Das Verfahren ist aber ebenso gültig für die verkürzte Form des FA-II Gens.

Das im Beispiel 4 beschriebene FA-II Gen, welches für die Aminosäuresequenz 1-223 von SEQ ID NO. 2 kodiert, wurde nach Standardverfahren (Sambrook, Fritsch, Maniatis, Molecular Cloning; A Laboratory Manual, 2nd Edition, Cold Spring Harbor, 1989) in den eukaryontischen Expressionsvektor pCDNA3 umkloniert (Invitrogen). Die Regulation der Expression geschieht durch den CMV Promotor/Enhancer und das BGH polyA Signal. Als Selektionsgen wird das Neo-Gen verwendet (unter Kontrolle der SV40 Expressionskassette).

Für die Herstellung einer stabilen, FA-II exprimierenden Zellinie wurden CHO Zellen verwendet. Dabei wurden 20 µg Lipofectamin (Gibco; in 750 µl MEM-alpha Medium) mit 10 µg DNA (in 750 µl MEM-alpha Medium) gemischt, 45 Minuten bei Raumtemperatur inkubiert und anschließend mit 6 ml MEM-alpha Medium verdünnt. Dieses Gemisch wurde für 6 Stunden auf 5 x 10⁶ CHO Zellen in T75 Zellkulturflaschen (Nunc) in MEM-alpha Medium (Gibco) gegeben. Die Inkubation erfolgte bei 37°C. Anschließend wurden die Zellen mit MEM-alpha/10% FKS (fötales Kälberserum) gewaschen und in frischem Medium für 48 Stunden bei 37°C kultiviert. Danach wurde durch Zugabe von 1 mg/ml Neomycin (G418, Boehringer Mannheim) der Selektionsdruck angelegt. Die überlebenden Zellen wurden mittls FACS (Becton Dickinson) als Einzelzellen in 96-er Well Zellkulturplatten (Nunc) mit frischem Medium kloniert und unter G418 Selektionsdruck weiter kultiviert, bis ein stabil transfizierter CHO Klon etabliert war.

Unter Verwendung von DHFR als Selektionsgen kann neben der Gewinnung einer stabil transfizierten CHO Zellinie zusätzlich eine Genamplifikation des FA-II Gens erreicht werden.

### Beispiel 6:

### Antikörperherstellung

Wie schon in Beispiel 5, wird hier exemplarisch die Antikörperherstellung mit der langen Variante des FA-II Proteins beschrieben. Das Verfahren ist ebenso gültig für die verkürzte Form des FA-II Proteins.

BALB/c Mäuse wurdem mit rekombinantem, humanem FA-II Protein mit der Aminosäuresequenz 1 - 223 von SEQ ID NO. 2 (hergestellt in CHO Zellen) intraperitoneal immunisiert. Die Erstimmunisierung erfolgte in komplettem Freund'schen Adjuvans und alle weiteren Immunisierungen wurden in inkomplettem Freund'schen Adjuvans durchgeführt. Die Dosis betrug 50 - 100 µg. Folgeimmunisierungen wurden in ca. 4-wöchigem Abstand durchgeführt, bis ein Serumtiter von 1:50000 erreicht ist.

Anschließend erfolgte die Immortalisierung der Milzzellen der immunisierten Tiere mit der Myelomzellinie P3XX63.Ag8.653. Die Fusion wurde nach Standardverfahren durchgeführt (J. Immunol. Methods 39 (1980), 285-308). Das Fusionsverhältnis Milzzellen zu Myelomzelle war dabei 1:1. Die Fusionsprodukte wurden auf 24-er Zellkulturschalen (Nunc) in HA-Medium auf RPMI/10% FKS Basis (Boehringer Mannheim) ausgesät. Positive Primärkulturen wurden 2 Wochen nach Fusion mittels FACS (Becton Dickinson) als Einzelzellen in 96-er Zellkulturplatten (Nunc) in RPMI/10% FKS kloniert.

Zur Gewinnung der monoklonalen Antikörper wurden die so erhaltenen Hybridomzellklone in vivo expandiert. Dazu wurden 5 x 10⁶ Hybridomzellen intraperitoneal in mit Pristan (Sigma Chemical Company) vorbehandelte Mäuse inokkuliert. Nach 10-21 Tagen wurden je Maus 2-3 ml Ascites entnommen und daraus der monoklonale Antikörper nach herkömmlichen Methoden gewonnen.

## Patentansprüche

1. Nucleinsäure, ausgewählt aus der Gruppe bestehend aus
(a) der in SEQ ID NO:1 dargestellten Nucleotidsäure oder einem Protein-codierenden Abschnitt davon,
(b) einer der Nukleinsäuren aus (a) im Rahmen der Degeneration des genetischen Codes entsprechenden Nucleotidsequenz,
(c) einer komplementären Nucleotidsäure hierzu,
(d) einer mit den Sequenzen aus (a) oder/und (b) unter stringenten Bedingungen hybridisierenden Antisense-Nudeotidsäure einer Länge von bis zu 50 Nucleotiden
mit der Maßgabe, daß die Nucleinsäure verschieden ist von den Nucleotidsäuren mit den Zugangsnummern W44613, W44574 und g1664579, angegeben in der EMBL EST Datenbank.

2. Vektor, **dadurch gekennzeichnet, daß** er mindestens eine Kopie einer Nucleinsäure nach Anspruch 1 oder einen Abschnitt davon enthält.

3. Zelle, **dadurch gekennzeichnet, daß** sie mit einer Nucleinsäure nach Anspruch 1 oder einem Vektor nach Anspruch 2 transformiert ist

4. Polypeptid, **dadurch gekennzeichnet, daß** es von einer Nucleinsäure nach Anspruch 1 codiert ist, wobei die Maßgabe von Anspruch 1 nicht zu berücksichtigen ist

5. Polypeptid nach Anspruch 4, **dadurch gekennzeichnet, daß** es
(a) die in SEQ ID NO:2 dargestellte Aminosäuresequenz von 1-223,
(b) die in SEQ ID NO:2 dargestellte Aminosäuresequenz von Aminosäure 59-223 oder
(c) die in SEQ ID NO:2 dargestellte Aminosäuresequenz von Aminosäure 1-40, 59-120 oder 205-223 aufweist.

6. Verwendung eines Polypeptids nach Anspruch 4 oder 5 oder von Fragmenten dieses Polypeptids als Immunogen zur Herstellung von Antikörpern.

7. Antikörper gegen ein Polypeptid nach Anspruch 4 oder 5.

8. Antikörper nach Anspruch 7, **dadurch gekennzeichnet, daß** er gegen das gesamte Polypeptid oder gegen eine Peptidsequenz entsprechend den Aminosäuren 1-40, 59-120 oder 2005-223 aus SEQ ID NO:2 gerichtet ist.

9. Pharmazeutische Zusammensetzung, **dadurch gekennzeichnet, daß** sie als aktive Komponente umfaßt:
(a) eine Nucleinsäure nach Anspruch 1, wobei die Maßgabe von Anspruch 1 nicht zu berücksichtigen ist,
(b) einen Vektor nach Anspruch 2,
(c) eine Zelle nach Anspruch 3,
(d) ein Polypeptid nach Anspruch 4 oder 5 oder/und
(e) einen Antikörper nach Anspruch 7 oder 8.

10. Verwendung einer Zusammensetzung nach Anspruch 9 zur Diagnostik von Erkrankungen, die mit Störungen des Zellzyklus, der Zellaktivierung, der Zellzyklusprogression, der DNA-Reparatur, Cytopenien, der Tumorgenese oder/und der Tumorprogression assoziiert sind, in einer aus einem Patienten stammenden Probe.

11. Verwendung einer Zusammensetzung nach Anspruch 9 zur Diagnostik einer Prädisposition für Erkrankungen, die mit Störungen des Zellzyklus, der Zellaktivierung, der Zellzyklusprogression, der DNA-Reparatur, Cytopenien, der Tumorgenese oder/und der Tumorprogression assoziiert sind, in einer aus einem Patienten stammenden Probe.

12. Verwendung einer Zusammensetzung nach Anspruch 9 zur Herstellung eines Mittels für die Diagnostik von Erkrankungen, die mit Störungen des Zellzyklus, der Zellaktivierung, der Zellzyklusprogression, der DNA-Reparatur, Cytopenien, der Tumorgenese oder/und der Tumorprogression assoziiert sind oder eines Mittels für die Diagnostik einer Prädisposition für solche Erkrankungen.

13. Verwendung einer Zusammensetzung nach Anspruch 9 zur Herstellung eines Mittels für die Therapie oder Prävention von Erkrankungen, die mit Störungen des Zellzyklus, der Zellaktivierung, der Zellzyklusprogression, der DNA-Reparatur, Cytopenien, der Tumorgenese oder/und der Tumorprogression assoziiert sind.

14. Verfahren zur Identifizierung von Effektoren eines Proteins nach Anspruch 4 oder 5, **dadurch gekennzeichnet, daß** man Zellen, die das Protein exprimieren, mit verschiedenen potentiellen Effektorsubstanzen in Kontakt bringt und die Zellen auf Veränderungen analysiert.

## Claims

1. Nucleic acid selected from the group comprising
(a) the nucleotide acid shown in SEQ ID NO.1 or a protein-coding section thereof,
(b) a nucleotide sequence corresponding to the nucleic acids from (a) within the scope of the degeneracy of the genetic code or
(c) a complementary nucleotide acid thereto,
(d) an antisense nucleotide acid having a length of up to 50 nucleotides which hybridizes with one of the sequences from (a) or/and (b) under stringent conditions
provided that the nucleic acid is different from the nucleotide acids with the accession numbers W44613, W44574 and g1664579 specified in the EMBL EST data bank.

2. Vector, wherein it contains at least one copy of a nucleic acid as claimed in claim 1 or a section thereof.

3. Cell, wherein it is transformed with a nucleic acid as claimed in claim 1 or with a vector as claimed in claim 2.

4. Polypeptide, wherein it is coded by a nucleic acid as claimed in claim 1 whereby the proviso of claim 1 can be disregarded.

5. Polypeptide as claimed in claim 4, wherein it has
(a) the amino acid sequence from 1-223 shown in SEQ ID NO. 2,
(b) the amino acid sequence from amino acid 59-223 shown in SEQ ID NO. 2 or
(c) the amino acid sequence from amino acid 1-40, 9-120 or 205-223 shown in SEQ ID NO.2.

6. Use of a polypeptide as claimed in claim 4 or 5 or of fragments of this polypeptide as an immunogen for the production of antibodies.

7. Antibody against a polypeptide as claimed in claim 4 or 5.

8. Antibody as claimed in claim 7, wherein it is directed against the entire polypeptide or against a peptide sequence corresponding to the amino acids 1-40, 59-120 or 2005-223 from SEQ ID NO. 2.

9. Pharmaceutical composition, wherein it comprises as the active component
(a) a nucleic acid as claimed in claim 1 whereby the proviso of claim 1 can be disregarded,
(b) a vector as claimed in claim 2,
(c) a cell as claimed in claim 3,
(d) a polypeptide as claimed in claim 4 or 5 or/and
(e) an antibody as claimed in claim 7 or 8.

10. Use of a composition as claimed in claim 9 for diagnosing diseases which are associated with disorders of the cell cycle, cell activation, cell cycle progression, DNA repair, cytopenias, tumorigenesis or/and tumour progression, in a sample derived from a patient.

11. Use of a composition as claimed in claim 9 for diagnosing a predisposition to diseases that are associated with disorders of the cell cycle, cell activation, cell cycle progression, DNA repair, cytopenias, tumorigenesis or/and tumour progression, in a sample derived from a patient.

12. Use of a composition as claimed in claim 9 for the production of an agent for diagnosing diseases that are associated with disorders of the cell cycle, cell activation, cell cycle progression, DNA repair, cytopenias, tumorigenesis or/and tumour progression or an agent for diagnosing a predisposition to such diseases.

13. Use of a composition as claimed in claim 9 for the production of an agent for the treatment or prevention of diseases that are associated with disorders of the cell cycle, cell activation, cell cycle progression, DNA repair, cytopenias, tumorigenesis or/and tumour progression.

14. Method for the identification of effectors of a protein as claimed in claim 4 or 5, wherein ells which express the protein are contacted with various potential effector substances and the cells are analysed for changes.

## Revendications

1. Acide nucléique choisi parmi le groupe constitué par :
(a) l'acide nucléique représenté par la SEQ ID NO:1 ou une portion de celui-ci codant une protéine,
(b) une des séquences nucléotidiques correspondant aux nucléotides de (a) dans le cadre de la dégénérescence du code génétique,
(c) un acide nucléique complémentaire
(d) un acide nucléotide antisens d'une longueur jusqu'à 50 nucléotides s'hybridant avec les séquences de (a) et/ou de (b) dans des conditions de stringence
à la condition que l'acide nucléique soit différent des acides nucléiques de numéro d'accès W44613, W44574 et g1664579, indiqués dans la banque de données EMBL EST.

2. Vecteur, **caractérisé en ce qu'**il contient au moins une copie d'un acide nucléique selon la revendication 1 ou une portion de celui-ci.

3. Cellule, **caractérisée en ce qu'**elle est transformée par un acide nucléique selon la revendication 1 ou un vecteur selon la revendication 2.

4. Polypeptide, **caractérisé en ce qu'**il est codé par un acide nucléique selon la revendication 1, pour lequel on ne tient pas compte de la condition négative de la revendication 1.

5. Polypeptide selon la revendication 4, **caractérisé en ce que** :
(a) il comporte la séquence d'acides aminés 1-223 de la SEQ ID NO:2
(b) il comporte la séquence d'acides aminés des acides aminés 59-223 de la SEQ ID NO:2 ou
(c) il comporte la séquence d'acides aminés des acides aminés 1-40, 59-120 ou 205-223 de la SEQ ID NO:2.

6. Utilisation d'un polypeptide selon la revendication 4 ou 5 ou de fragments de ce polypeptide comme immunogène pour la production d'anticorps.

7. Anticorps dirigés contre un polypeptide selon la revendication 4 ou 5.

8. Anticorps selon la revendication 7, **caractérisé en ce qu'**il est dirigé contre le polypeptide entier ou contre une séquence peptidique correspondant aux acides aminés 1-40, 59-120 ou 2005-223 de SEQ ID NO:2.

9. Composition pharmaceutique, **caractérisée en ce qu'**elle contient comme composants actifs :
(a) un acide nucléique selon la revendication 1, pour lequel on ne tient pas compte de la condition négative de la revendication 1.
(b) un vecteur selon la revendication 2,
(c) une cellule selon la revendication 3,
(d) un polypeptide selon la revendication 4 et 5 et/ou
(e) un anticorps selon la revendication 7 ou 8.

10. Utilisation d'une composition selon la revendication 9 pour le diagnostic de maladies, qui sont associées aux troubles du cycle cellulaire, de l'activation cellulaire, de la progression du cycle cellulaire, de la réparation de l'ADN, des cytopénies, de la genèse tumorale et/ou de la progression tumorale, dans un échantillon provenant d'un patient.

11. Utilisation d'une composition selon la revendication 9 pour le diagnostic d'une prédisposition à des maladies, qui sont associées aux troubles du cycle cellulaire, de l'activation cellulaire, de la progression du cycle cellulaire, de la réparation de l'ADN, des cytopénies, de la genèse tumorale et/ou de la progression tumorale, dans un échantillon provenant d'un patient.

12. Utilisation d'une composition selon la revendication 9 pour la production d'un moyen de diagnostic de maladies qui sont associées aux troubles du cycle cellulaire, de l'activation cellulaire, de la progression du cycle cellulaire, de la réparation de l'ADN, des cytopénies, de la genèse tumorale et/ou de la progression tumorale ou d'un moyen de diagnostic d'une prédisposition à de telles maladies.

13. Utilisation d'une composition selon la revendication 9 pour la production d'un moyen pour la thérapie ou la prévention de maladies, qui sont associées aux troubles du cycle cellulaire, de l'activation cellulaire, de la progression du cycle cellulaire, de la réparation de l'ADN, des cytopénies, de la genèse tumorale et/ou de la progression tumorale.

14. Procédé d'identification d'effecteurs d'une protéine selon la revendication 4 ou 5, **caractérisé en ce que** l'on met en contact les cellules qui expriment la protéine avec différentes substances effectrices potentielles et que l'on analyse les modifications de cellules.
